# EUROPEAN PATENT APPLICATION

(11) **EP 0 863 150 A1**
(43) Date of publication of application: **09.09.1998**
(21) Application number: 98102059.7
(22) Date of filing: 06.02.1998
(51) Int. Cl.: C07K 14/00, C07D 473/18, C07D 473/32, C07D 473/40

(54) **A method for the preparation of nucleic acid binding compound**

(30) Priority: 08.02.1997 EP 97102028
(71) Applicant: BOEHRINGER MANNHEIM GMBH, 68305 Mannheim (DE)
(72) Inventor: Batz, Hans-Georg Dr., 82327 Tutzing (DE); Hansen, Henrik Frydenlund, 2660 Rodovre (DK); Orum, Henrik Dr., 3500 Vaerlose (DK); Koch, Troels Dr., 2300 Kobenhaven S. (DK); Kofoed, Thomas, 5260 Odense S. (DK)

(57) **Abstract**

A method of the preparation of a nucleic acid binding compound is disclosed wherein a first protecting group removable by a medium strong base and a second protecting group removable by a medium strong acid are used. The synthesis is thereby is increased in recovery yield of a nucleic acid binding compound prepared.

## Description

This invention is directed to a method for the preparation of the nucleic acid binding compound using specific protection/deprotection strategy, compounds used for the synthesis and a method for preparation of selectively double-labeled nucleic acid binding compounds.

### Background of the invention

Recently, compounds were developed that are binding nucleic acids via nucleo base-pairing in a considerably stable way (WO 92/20702). Such compounds are prepared in a method based on subsequent addition of monomers to a solid phase-bound starting functional group. In the synthesis of peptide nucleic acid (PNA), monomers are used containing an amino group which is protected by the Boc group. The monomers contain a free carboxylic acid group and are reacted with an amino group on the solid phase which is either the starting functional group or is set free by deprotecting a monomer attached earlier in the synthesis. After coupling, the Boc protection group of the monomer is removed from the growing chain by a medium strong acid, for example, trifluoroacetic acid.

In WO 93/12129 it is proposed to use the Z group to protect cytosine, adenine and guanine during this synthesis. The said groups are removed from the amino groups on the exocyclic amines by very strong acids, for example, trifluoromethanesulphonic acid.

In WO 95/17403 the preparation of PNA by selectively carbamate protected guanine monomers is described. The amino group on the backbone is protected by the Boc group.

In EP-0 672 677 there is described a method for synthesis nucleic acid analogues, wherein the protecting group on the amino group at the backbone is cleaved by either medium strength base (Fmoc) or by weak acids (Tr) and the protecting groups at exocyclic amino groups of the bases are removed by either weak acids (Tr) or medium strong bases (Acyl).

These methods proposed up to now are suffering from several drawbacks. They are either based on complicated and expensive monomer syntheses which yield monomers not optimal for PNA synthesis, or based on synthesis strategies which, although suited for PNA synthesis, yield low recoverage of the crude material which is contaminated with high amounts of residuals. The commonly used carbamate protecting groups (Z) for amino groups at the bases is cleavable by strong acids, which are harsh conditions.

### Summary of the invention

Subject of the invention is a method for the preparation of a nucleic acid binding compound comprising the steps
a) preparing a protected nucleic acid binding compound, comprising a plurality of ligands, capable of binding to nucleobases of a nucleic acid via hydrogen bonds bound to a backbone, at least one of said ligands containing a primary or secondary amino group protected by a first protecting group P1 which is removable by a medium strong base, said backbone further comprising a protected amino group of the formula O: 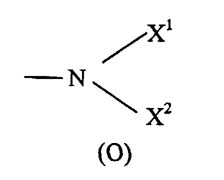 wherein X¹ is selected from the group consisting of hydrogen, (C₁ - C₃) alkyl and an amino-protecting group ,P2, preferably removable by a medium strong acid and X² is an amino protecting group, P3, removable by a medium strong acid;
b) removing said medium strong acid removable group(s), which is P3 and, if present P2; and
c) removing said medium strong base removable group P1.

The object of the invention is to provide a synthesis strategy wherein an orthogonal protecting group strategy is applied.

Preferably only one of P2 and P3 is a protecting group.

It is a further object of the present invention to improve monomer and oligomer synthesis. Thus, the monomer syntheses are straight forward and oligomerisation of these in the PNA synthesis provides crude material with higher purity and in higher yield.

A further subject of the present invention is to provide an easy synthesis of oligomers containing monomers comprising cytosine (C), adenine (A), guanine (G) or/and diaminopurine (D).

A further object of this invention is to provide a new molecular tool to prepare molecules which are sensitive to strong acids, e.g. PNA/DNA chimeras and conjugates such as those containing disulfides. Also, the orthogonal protection strategy offers the possibility to operate with several different protection groups which can be selectively removed and can be used to prepare selectively double labeled oligomers.

### Brief description of the drawings

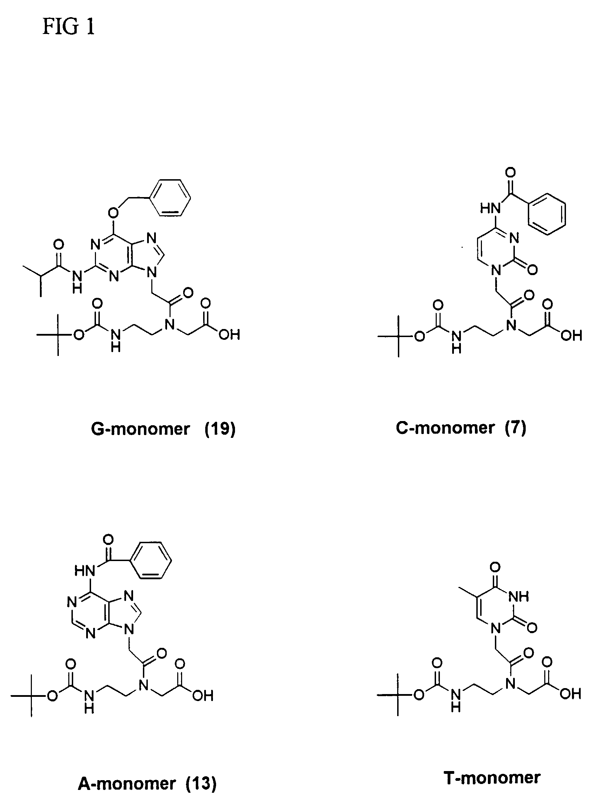

FIG 1 shows PNA monomers for the nucleobases A, C, G and T used in the examples following the new synthesis strategy.

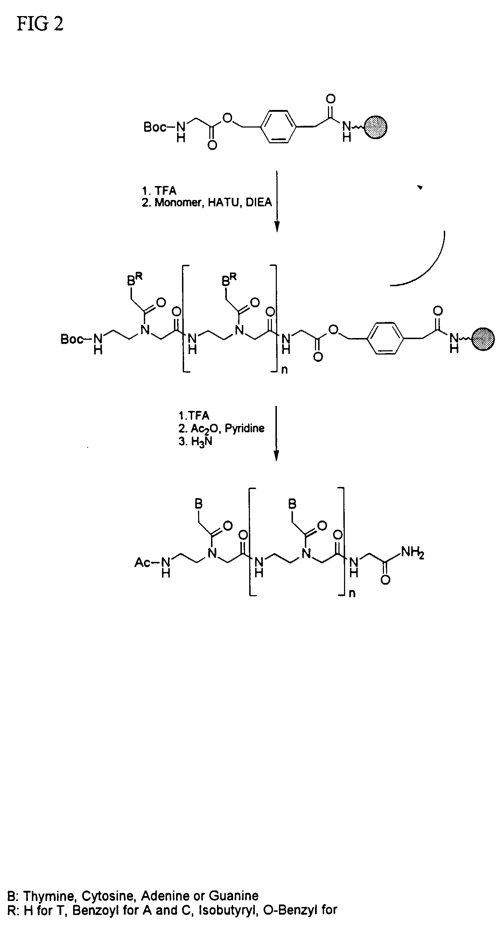

FIG 2 shows schematically the extention reaction of a solid phase bound linker by PNA monomers and the cleavage of the oligomer from the solid support.

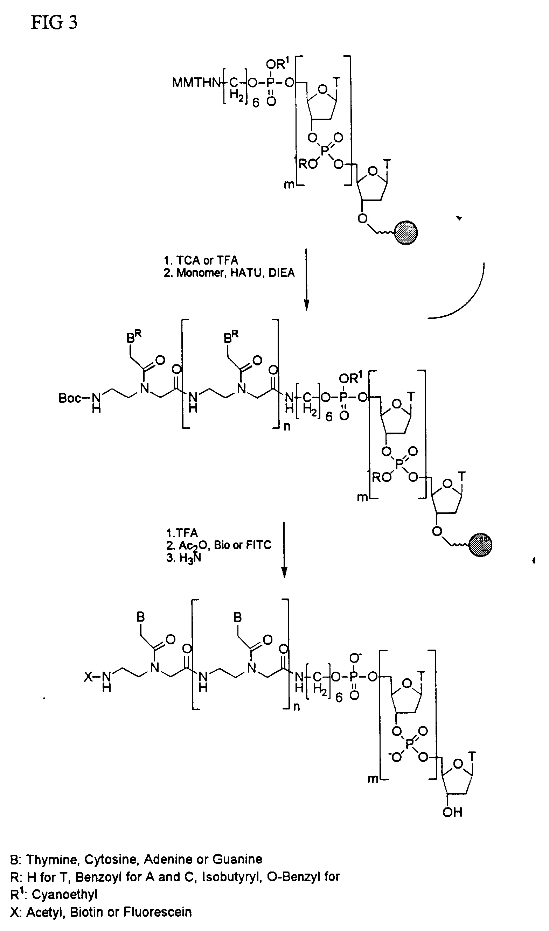

FIG 3 shows schematically the extension reaction of a solid support bound oligonucleotide by PNA monomers and the cleavage of the resulting chimeric molecule from this support.

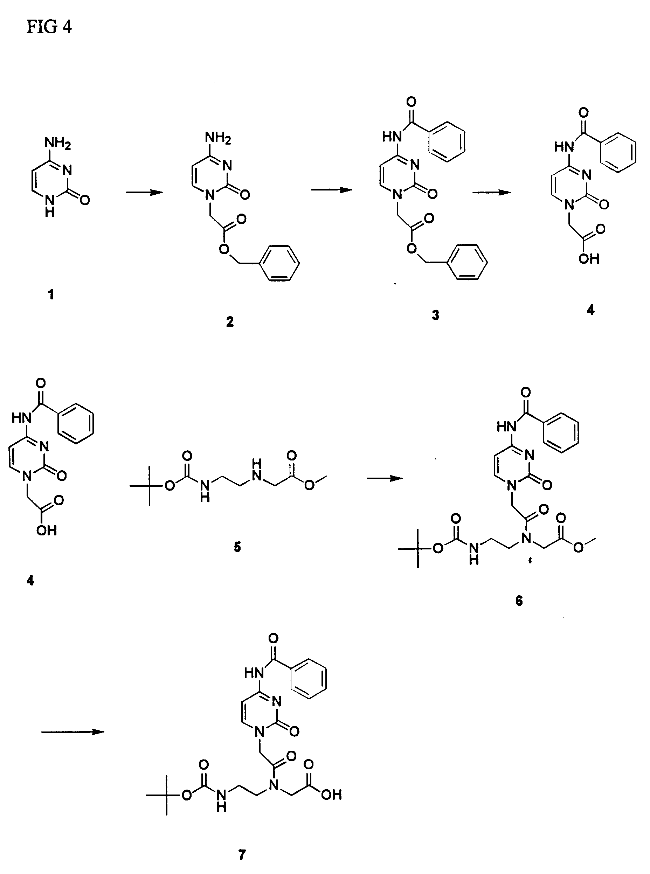

FIG 4 shows the route of synthesis for the protected C-monomer.

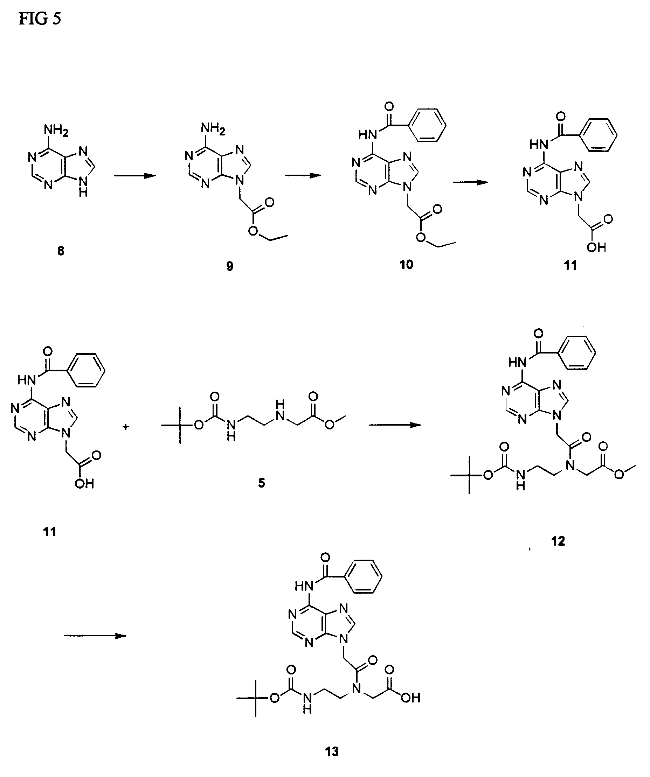

FIG 5 shows the route of synthesis for the protected A-monomer.

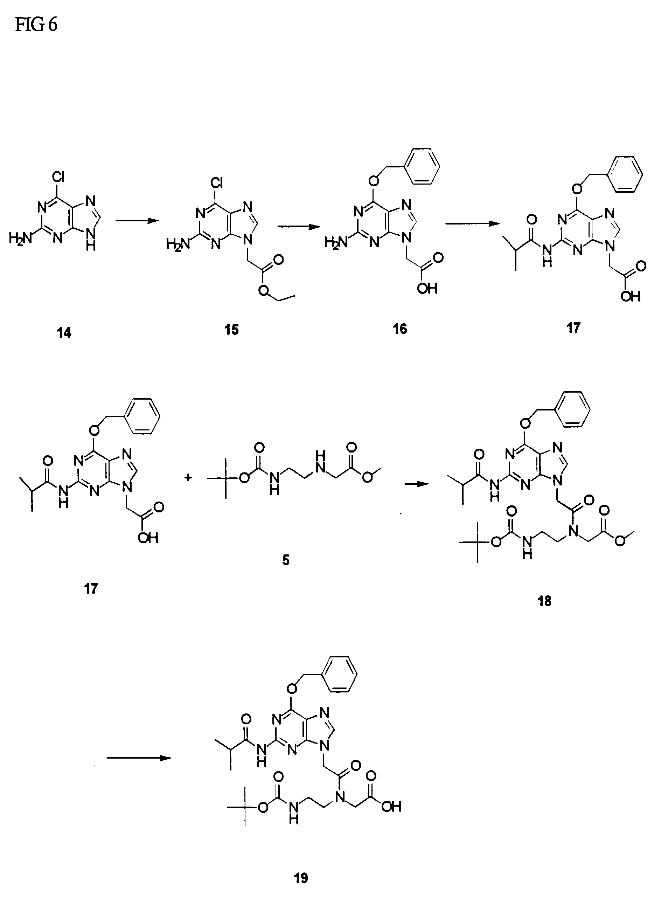

FIG 6 shows the route of synthesis for the protected G-monomer.

FIG 7 shows the route of synthesis for the protected D-monomer. D means diaminopurine.

FIG 8 shows a flowscheme of the preparation of a protected T-lysine monomer.

### Definitions

A protecting group according to the present invention is a chemical group that prevents the functional group to which it is attached from participating in a chemical reaction which is not intended. The protecting group can be removed from this functional group without destroying the nucleic acid binding properties of the nucleic acid binding compound and without non-intended removal of backbone units from the nucleic acid binding compound. In this invention focus is laid on protecting groups protecting amino groups of monomers during the synthesis of an oligomer from monomers. Examples of such protecting groups are P1, P2 and P3.

Nucleic acid binding compounds according to the present invention are molecules capable of binding to nucleic acids via base-pairing. Such base-pairing usually occurs by hydrogen bonding between complementary nucleo bases, like A base pairing with T as well as C base paring with G. Nucleic acid binding compounds can principally bind to nucleic acids in two ways. In a first case one strand of the nucleic acid binding compound binds to a selected region of one strand of a nucleic acid thereby forming a double helix strand, or duplex; or two molecules of the nucleic acid binding compound form a complex with the selected region on the nucleic acid, thereby forming a triple helix strand, or triplex. Such nucleic acid binding compounds may be known-in-the-art, for example from WO 92/20702. Not only PNA with identically repeating backbone moieties are encompassed by the present invention, but also nucleic acid binding compounds, wherein the backbone is made up by different monomeric backbone subunits. Such compounds are described, for example, in WO 95/14706, the content of which is hereby incorporated for defining the nucleic acid binding compound structure. Further encompassed are compounds as disclosed in EP-A-0 672 677, wherein there are disclosed mixed structures of peptide bonded monomers and oligonucleotide subunits. Further encompassed are compounds as disclosed in WO 96/20212 and EP-A-0 700 928.

Ligands are preferentially selected among the group consisting of hydrogen, phenyl, fused aromatic moieties, heterocyclic moieties, naturally occurring nucleobases and non-naturally occurring nucleobases as well as reporter groups. Naturally occurring nucleobases are for example the main nucleobases like thymine, cytosine, adenine and guanine or rare bases like inosine, 5-methylcytosine or thiouracil. An example for a non-naturally occurring nucleobase is 7-deaza-guanine. An example for a fused aromatic moiety is naphthol. A heterocyclic moiety is pyridine. Reporter groups are moieties that can be detected, like fluorescent compounds, for example fluorescein, or moieties that can be recognized by another molecular entity, like haptens which can be recognized by an antibody raised against this hapten. In order to achieve binding of the nucleic acid binding compound to the nucleic acid, the nucleic acid binding compound generally will comprise a sufficient number of nucleobases as ligands for base pairing to nucleobases of the nucleic acid, for example at least 6 nucleobases. For example, at the end of the backbone having the nucleobases attached, the backbone may contain ligands other than nucleobases.

The backbone of the nucleic acid binding compound is preferably a moiety containing subunits arranged in a linear manner, to which subunits the ligands may be bound at different sites. The backbone of a nucleic acid binding compound according to the present invention contains at least one primary or secondary amino group at one of the termini. However, preferably the backbone contains additional amino groups, especially amino groups of one monomeric subunit used for attachment of this subunit to another subunit, preferably via a carboxylic acid group of this other subunit. The subunits of said backbone, without taking into consideration the ligands, are preferably identical. However, subunits containing amino and carboxyl groups can also be mixed with subunits containing other functional groups for attachment.

A label is generally known to a man skilled in the art as being a group which is detectable or can be made detectable for determining the presence of the nucleic acid binding compound. The label should therefore be discriminable either photometrically or by other means, like by immunoreactive properties from the rest of the nucleic acid binding compound and any compounds to be determined, like the nucleic acid. Well-known labels are fluorescent labels, like fluoresceine, electrochemoluminescent labels, like ruthenium complexes, biotin or digoxigenin.

Crude material in the present invention is the nucleic acid binding compound directly eluted from the solid phase, on which it was synthesized, without any further workup. The yield of such crude material is calculated from the theoretical yield, calculated from the amount of monomers used, compared to the amount of nucleic binding compound contained in the eluate.

### Detailed description of the invention

Because the reaction of carboxylic acid (COOH) groups with amino (NH₂) groups is well known to a man skilled in the art, nucleic acid binding compounds, wherein the polymeric backbone contains amide bonds between carboxylic acid and amino groups of adjacent monomeric subunits (monomers) are recently becoming important. The general reaction is well-known from the synthesis of the polymerization of amino acids to create peptides, which for example can be a specific epitope part of a protein. However, peptide syntheses generally do not take into considering any base-pairing to nucleic acids. Base-pairing to nucleic acids can be accomplished by having attached to a peptide backbone, or a pseudo peptide backbone, a number of ligands capable of binding to nucleobases on a nucleic acid. Such interstrand binding is often provided via hydrogen bonds, which require either reactive amino groups (proton donors) and oxygen or nitrogen (proton acceptors).

Nucleic acid binding compounds that can be produced according to the present invention are exemplified in WO 92/20702. The preferred peptide nucleic acids are characterized in that they comprise ligands linked to the pseudo peptide backbone via an aza nitrogen atom. It is most preferred that besides this aza nitrogen atom, at least some of the monomeric building blocks are connected to each other using amide bonds (-CONH-). Preferred peptide nucleic acids are shown in formula I: 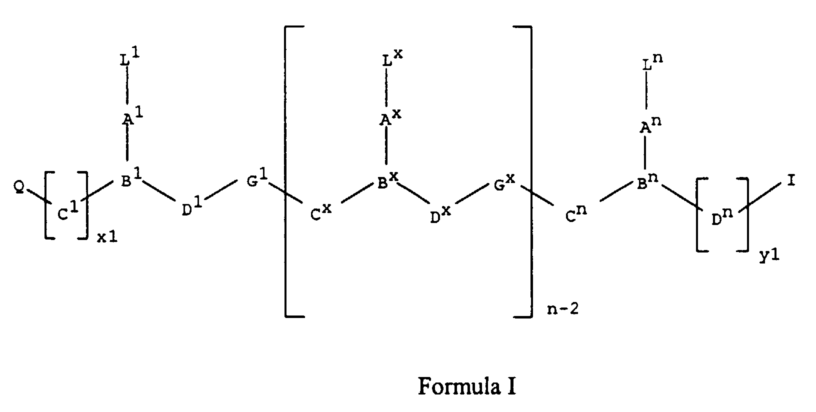 wherein
n is an integer of from at least 3,
x is an integer of from 2 to n-1,
each of L¹-Lⁿ is a ligand independently selected from the group consisting of hydrogen, hydroxyl, (C₁-C₄)alkanoyl, naturally occurring nucleobases, non-naturally occurring nucleobases, aromatic moieties, DNA intercalators, nucleobase-binding groups, heterocyclic moieties, reporter ligands and chelating moieties, at least one of L¹⁻ Lⁿ containing a primary or secondary amino group;
each of C¹-Cⁿ is (CR⁶R⁷)y (preferably CR⁶R⁷, CHR⁶CHR⁷ or CR⁶R⁷CH₂) where R⁶ is hydrogen and R⁷ is selected from the group consisting of the side chains of naturally occurring alpha amino acids, or R⁶ and R⁷ are independently selected from the group consisting of hydrogen, (C₁-C₆)alkyl, aryl, aralkyl, heteroaryl, hydroxyl, (C₁-C₆)alkoxy, (C₁-C₆)alkylthio, NR³R⁴ and SR⁵, where R³ and R⁴ are as defined below, and R⁵ is hydrogen, (C₁-C₆)alkyl, hydroxyl, (C₁-C₆)alkoxy, or (C₁-C₆)alkylthio-substituted (C₁-C₆)alkyl or R⁶ and R⁷ taken together complete an alicyclic or heterocyclic system; or C¹-Cⁿ is CO, CS, CNR³;
each of D¹-Dⁿ is (CR⁶R⁷)_{z} (preferably CR⁶R⁷, CHR⁶CHR⁷ or CH₂CR⁶R⁷) where R⁶ and R⁷ are as defined above;
each of y and z is zero or an integer from 1 to 10, the sum y + z being at least 2, preferably greater than 2, but not more than 10;
each of G¹-Gⁿ⁻¹ is -NR³CO-, -NR³CS-, NR³SO- or -NR³SO₂-, in either orientation, where R³ is as defined below;
each of A¹-Aⁿ and B¹-Bⁿ are selected such that:
   (a) A¹-Aⁿ is a group of formula (I/A), (I/B), (I/C) or (I/D), and B¹-Bⁿ is N or R³N⁺; or
   (b) A¹-Aⁿ is a group of formula (I/D) and B¹-Bⁿ is CH; 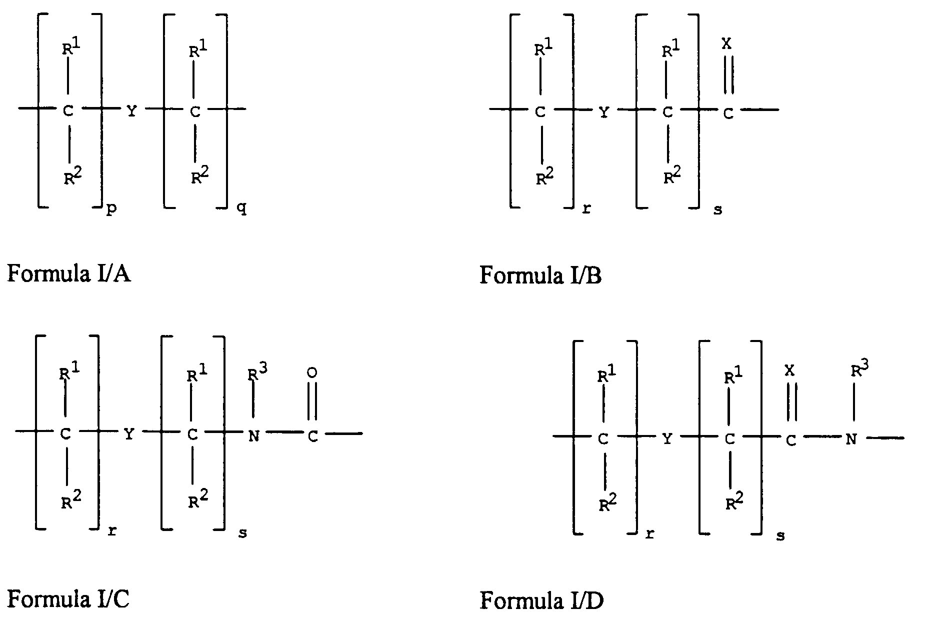 wherein:
      X is O, S, Se, NR³, CH₂ or C(CH₃)₂;
      Y is a single bond, O, S or NR⁴;
      each of p and q is zero or an integer from 1 to 5, (the sum p+q being preferably not more than 5);
      each of r and s is zero or an integer from 1 to 5, (the sum r+s being preferably not more than 5);
      each R¹ and R² is independently selected from the group consisting of hydrogen, (C₁-C₄)alkyl which may be hydroxyl- or (C₁-C₄)alkoxy- or (C₁-C₄)alkylthio-substituted, hydroxyl, (C₁-C₄)alkoxy, (C₁-C₄)alkylthio, amino and halogen; and
      each R³ and R⁴ is independently selected from the group consisting of hydrogen, (C₁-C₄)alkyl, hydroxyl- or alkoxy- or alkylthio-substituted (C₁-C₄)alkyl, hydroxyl, (C₁-C₆)-alkoxy, (C₁-C₆)-alkylthio and amino;
      Q and I are independently selected from the group consisting of NH₂, CONH₂, COOH, hydrogen, (C₁-C₆)-alkyl, O-(C₁-C₆)-alkyl, amino protected by a amino protecting groups, reporter ligands, intercalators, chelators, peptides, proteins, carbohydrates, lipids, steroids, nucleosides, nucleotides, nucleotide diphosphates, nucleotide triphosphates, oligonucleotides, including both oligoribonucleotides and oligodeoxyribonucleotides, oligonucleosides and soluble and non-soluble polymers as well as nucleic acid binding moieties and
      each of x1 and y1 is an integer of from 0 to 10.

Most preferred nucleic acid binding compounds comprise at least one monomeric subunit of the general formula II: 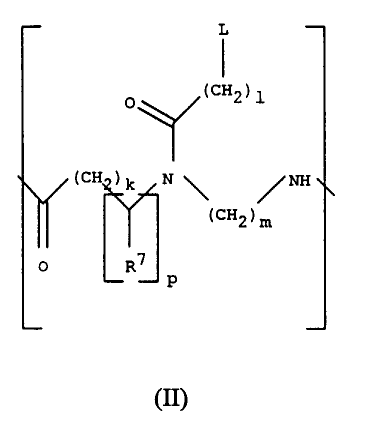 wherein
L is a ligand as defined above for L¹-Lⁿ,
k, l and m is independently zero or an integer from 1 to 5,
p is zero or 1, and
R⁷ is selected from the group consisting of hydrogen and the side chains of naturally occurring alpha amino acids.

A protected nucleic acid binding compound according to the present invention is a nucleic acid binding compound, wherein a primary or secondary amine of at least one of said ligands is protected by a first group P1 removable by a medium strong base, and wherein the backbone comprises a protected amino group of the formula O: 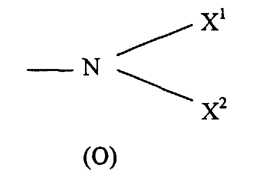 wherein
- X¹: is selected from the group consisting of hydrogen, (C₁ - C₃) alkyl and an amino-protecting group P2, preferably removable by a medium strong acid and
- X²: is an amino protecting group P3 removable by a medium strong acid.

Preferably these protected compounds are attached to a solid phase, for example to a non-soluble polymer, most preferably defined such that one terminus of the protected nucleic acid binding compound is bound to the solid phase, i.e. I or Q in formula I is a non-soluble polymer, for example polystyrene containing functional amino groups to which the compound is bound via a carbonyl group between Dⁿ and I or C ¹ and Q. Such binding to solid phases are well known and applied in the prior art for PNA. These compounds may contain further protected functional groups.

Further, the invention is directed to the preparation of said nucleic acid binding compounds including the preparation of said protected nucleic acid binding compound. During this chemical synthesis the protection of an amino group on the monomeric backbone building block is necessary. During the attachment of said monomeric building block the protection group remains attached, but after the attachment of the monomer building block the protecting group is removed and the backbone is further extended, for example, by attaching a subsequent monomer building block. During this synthesis, the monomeric building blocks as disclosed in WO 92/20702 can be used for any monomeric compounds having ligands attached which do not contain any free primary or secondary amino groups. In order to achieve maximum efficiency, it is preferred, that the protecting groups at the backbone, for example within formula I are selected to be the same as in the monomers having a ligand containing a primary or secondary amino group protected by the first protecting group removable by the medium strong base. Therefore, at least one of the monomers has one of the general formulae IIIa, IIIb, and IIIc: 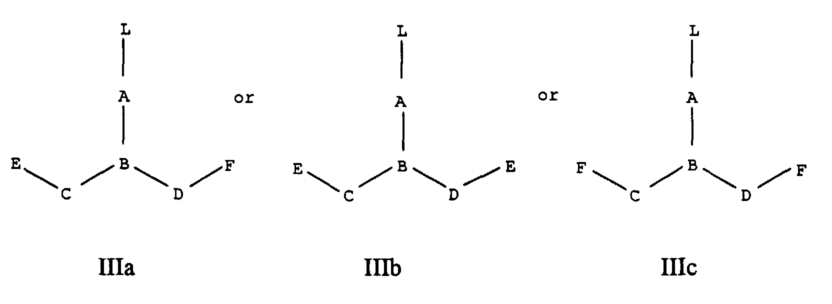 wherein
A is chosen from the definitions of A¹-Aⁿ in formula I,
B is chosen from the definitions of B¹-Bⁿ in formula I,
C is chosen from the definitions of C¹-Cⁿ in formula I,
D is chosen from the definitions of D¹-Dⁿ in formula I,
E is COOH, CSOH, SOOH, SO₂OH or an activated derivative thereof for example an activated ester like a N-hydroxyl succinimid ester,
F is a moiety of formula O, and
L is a ligand containing a primary or secondary amino group preferentially protected by a first protecting group removable by a medium strong base.

In the chemical synthesis of the protected nucleic acid binding compound E, or activated E, is reacted with a solid phase, or with the growing chain of the nucleic acid binding compound to be prepared capable of reacting with said reactive group E, thereby attaching said monomeric building block to the solid phase or with the growing chain of the nucleic acid binding compound to be prepared. Preferably all groups other than the groups intended to react with said reactive group are protected against such reaction.

After this reaction, and if the nucleic acid binding compound to be prepared contains more monomeric units than presently attached, the backbone amino protecting group(s) is (are) removed using a medium strong acid. Preferred are protecting groups of the urethane type. Such groups are generally known from peptide synthesis as well as from the synthesis as disclosed in WO 92/20702. Useful amino protecting groups are those which largely fulfill the following requirements: (1) stability to mild acids (not significantly attacked by carboxyl groups); (2) stability to mild bases or nucleophiles (not significantly attacked by the amino group in question); (3) resistance to acylation (not significantly attacked by activated carboxylic acids); and (4) the protecting group must be close to quantitatively removable, without serious side reactions. A preferred species of such protecting group is the tert.-butyl carbamate (Boc) group (Carpino, J. Am. Chem. Soc., 1957, 79, 4427; McKay, et al., J. Am. Chem. Soc., 1957, 79, 4686; Anderson, et al., J. Am. Chem. Soc., 1957, 79, 6180). Other suitable carbamates are: 1-methyl-1-phenylethyl carbamate, 1-methyl-1-(4-biphenylyl)ethyl carbamate, 1-methylcyclobutyl carbamate, allyl carbamate, cinnamyl carbamate, 8-quinolyl carbamate, 5-benzisoxazolylmethyl carbamate, N-pivaloyloxymethyl, N,N 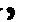 -isopropylidene and N-benzylidene (Greene & Wuts, "Protecting groups in organic synthesis", John Wiley & sons, 1991). Also preferred compounds are 1-methylcyclohexyl carbamate, 1-methyl-1-cyclopropyl carbamate (Brady, et al., J. Org. Chem., 1977, 143, 42), 1,1-diphenylmethyl carbamate, 1,1-di(4-methylphenyl)methyl carbamate, 1,1-dimethylpropenyl carbamate (Sieber & Iselin, Helv. Chim. Act., 1968, 614, 51), 1-adamantyl carbamate (J. Am. Chem. Soc., 1966, 79, 1988).

Medium strong acids according to the present invention have generally a pKₐ of between 2 and -4, preferably between 2 and - 2. Such medium strong acids are, for example, trifluoroacetic acid (TFA) and trichloroacetic acid (TCA). In order to remove the protecting group from the intermediate nucleic acid binding compound these are preferably treated such that the resin (solid phase) linked oligomer is treated for a total of 5-10 min with a preferred acid. The acid treatment is preferentially done by 2 consecutive treatments of a total of 5-10 min. The resin is thoroughly washed with organic solvents before the coupling step. Under such conditions, the protecting group(s) P1 are preferably not removed from the primary or secondary amino group(s) of the ligand.

Such conditions are, for example, described in WO 92/20702.

Within formula O it may be preferred to choose only one of X¹ and X² to be a protecting group (for example for sterical reasons). The other will be selected from the group of moieties not essentially inhibiting binding of X² to the nitrogen atom. Such other groups will preferably be selected from hydrogen and lower alkyl groups (C₁-C₆).

The protecting groups P1 removable by a medium strong base, protects primary or secondary amino groups of the ligand L from reacting with monomers designed for chain elongation for example by reacting with a carboxylic acid group of said monomer. Such primary or secondary amino groups are preferably the exocyclic amino groups on nucleobases. Preferred groups P1 are shown in formula IV: 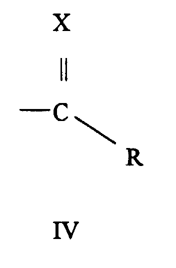 wherein X is O or S,and
R is selected from straight or branched chain (C₁ - C₆)-alkyl, which may be unsubstituted or substituted by C₁-C₄-alkoxy or substituted aromatic moieties, and aromatic moieties containing from 6 to 10 aromatic carbon atoms, which may be unsubstituted or substituted by C₁-C₄-alkyl, C₁-C₄-alkoxy, halogen (preferred Cl) or nitro.

Most preferred groups as represented by formula IV are acyl groups, especially preferred acetyl, isobutanoyl, benzoyl, phenoxyacetyl, 4-(t-butyl)-benzoyl, 4(t-butyl)-phenoxyacetyl and 4-(methoxy)-benzoyl.

Medium strong bases according to the present invention have generally a pKa of between 8 and 15, preferably between 8 and 12. Such medium strong bases are, for example, ammonia, methylamine, ethylamine, piperidine and dimethylamine each in aqueous/organic solutions.

Removal of the ligand protection group(s) P1 is performed by base treatment of the protected units in a time period ranging from a few minutes to several hours. The necessary deprotection time is determined by the activity of the medium strength base. When ammonia is used to deprotect the protected ligand the conditions will normally include treatment over night at 60 °C. Alkylated amines will normally deprotect faster and at a lower temperature. The amines will either be dissolved in water or in organic solvents, preferentially alkohols. If the oligomer is not cleaved from the resin, and only specific ligand protecting groups are removed, the solution (deprotecting mixture) is removed by filtration and further manipulations of the resin linked oligo can be performed. If the oligomer is cleaved from the resin during the base treatment, the deprotection mixture containing the oligomer is washed off from the resin and solvent can be removed by evaporation.

A primary amino group is the group -NH₂. A secondary amino group is the group -NH-. Preferred ligands containing primary amino groups, protected by preferentially base labile protecting groups, are adenine (exocyclic amino group in position 6), guanine (exocyclic amino group in position 2, cytosine (exocyclic amino group at position 4), and diaminopurine (one or both of the exocyclic amino groups in position 2 and 6). For guanine, it is also possible to protect the hydroxyl group in position 6 by the first set of alkylgroups mentioned above or by another appropriate oxygen protecting group, like O-benzyl.

Monomers of formulae IIIa-c are preferentially prepared by a convergent synthesis strategy, thus, the protected backbone unit and the protected ligands (e.g. bases) are first prepared by separate routes and then linked together. The backbone is prepared by reacting Boc-protected amino-acetaldehyde with a protected α-amino acid, e.g. alkyl glycinate. The bases are preferentially first alkylated with an α-halo-acetic acid derivative and then protected at the reactive positions. The protected and alkylated ligand (base) is then coupled via an amide bond to the central secondary amine of the backbone unit.

When having attached as many monomeric subunits to the solid phase as intended or required, the final protected nucleic acid binding compound is ready. It will contain the amino groups, especially the amino groups in the ligand, in protected form. In most cases this protected compound will not have the desired nucleic acid binding properties. Therefore, in order to prepare the final nucleic acid binding compound, it may be necessary to remove the protecting groups. In order to remove the protecting group P1, the compound is subjected to a treatment with a medium strong base.

If the nucleic acid binding compound was synthesized on a solid phase, it will be desired to cleave the compound from the solid phase on which it was synthesized. The conditions for this cleavage will depend upon the linker used for attaching the first monomer to the solid phase. Such binding and cleavage is also disclosed in WO92/20702.

Due to the orthogonal protecting strategy the present invention further provides a method to protect different ligands within one nucleic acid binding compound by different protecting groups. The method allows the preparation of labeled ligands at specific and defined positions within one sequence. The nucleic acid binding compound in protected form is prepared by using, within the chemical synthesis, a first monomeric backbone unit having bound a ligand with a protected primary or secondary amino group, and then a subsequent monomeric backbone unit bearing a ligand, which may be the same or, preferably, different from the ligand of the first monomeric backbone unit. If the subsequent attached monomeric unit contains a primary or secondary amino group protected by a protecting group which can be removed under different conditions by which the first protecting group is removed, this protecting group can be removed selectively.

In a first embodiment of this method of the invention the first protecting group P1, located on the first monomeric base unit, is removable by a medium strong base, as disclosed above, while the second group P1 located on a second monomeric base unit is removable by a strong acid. The amino protecting group on the backbone is removable by a medium strong acid for both monomers.

In a second embodiment of the invention the first protecting group P1, located on the base of a first monomeric base unit, is removable by a medium strong base, as disclosed above, while the second group located on the base of the second monomeric unit is removable by an other base. An example of the latter is the Fmoc group which is removable by medium strength sterically hindered bases, such as piperidine, and 1,8-diazabicyclo(5.4.0)undec-7-ene (DBU). The amino protecting group on the backbone is removable by a medium strong acid for both monomers. This protection group combination can also be composed so that the Fmoc group is used to protect the primary amine on the backbone, and the bases are protected by a combination of a group removable by medium strength base and a group removable with medium strength acid, as defined above.

In yet another embodiment of the invention the first protecting group P1, located on the first monomeric base unit, is removable by a medium strong base, as disclosed above, while the second group located on a second monomeric base unit is removable by a weak acid. Examples of the latter could be triphenylmethyl (Trt), mono-or-dimethoxy-triphenylmethyl (Mmt, Dmt). The amino protecting group on the backbone could then be Fmoc. In this embodiment the combination could also be composed by having the protecting group removable by weak acids to protect the primary amine on the backbone, and the bases could be protected by a combination of groups removable by a medium strength base and the Fmoc group.

Weak acids are defined to have a pKa of between 2-6.

There may be further monomeric backbone units protected by further different protecting groups selectively or not selectively removable. All differently ligand protected monomeric backbone units can be located at any position within the protected nucleic acid binding compound, depending upon the nucleobase sequence required for the specific purpose, like for complementarity to the nucleic acid to be determined.

Using such nucleic acid binding compounds in protected form having selectively cleavable protecting groups allows the easy preparation of differently labeled nucleic acid binding compounds. In such a method, the nucleic acid binding compound in protected form is incubated under conditions for removing selectively only one of said first and second protecting groups, and not the amino protecting group at the backbone. Preferably, under these conditions there will not occur any cleavage of the nucleic acid binding compound from the solid phase to which it may be bound. In the preferred case of the second group being a protecting group removable by weak acids, the nucleic acid binding compound in protected form is contacted with such a weak acid, for example mono-/or-di-/or-tri-chloro acetic acid, The now unprotected functional group, for example a hydroxyl group or an exocyclic amino group on the ligand is then reacted with a reactive species of the first label to be attached to this group.

In a next step, the first protecting group of any of the other ligands is removed. In the preferred case this will be made by applying to the nucleic acid binding compound in protected form a medium strong base, thereby setting free the primary or secondary amino group at that ligand, while not affecting the binding of the previously attached label group, the amino protecting group X¹ and X² on the backbone, and if the oligomer is still bound to the solid support, the attachment to the solid phase is also left untouched.

The now available primary or secondary amino group at the ligand is then reacted with a reactive species of the second label. The now bis-labeled protected nucleic acid binding compound may then be totally deprotected by removing any further protecting groups, like X¹ and X², and if the oligomer is still bound to the solid support the oligomer is removed from the support. The amino group from which the protecting groups are removed, typically on the backbone, can further be used to attach a third or further label by reacting this group with reactive species of that label. Thereafter, if the oligomer is bound to the solid support, the compound may be removed from the solid phase. This strategy may be applied to as many labels as functional groups as hydroxyl or amino groups are available within the backbone and/or the ligands. It is apparent to a man skilled in the art that the selectivity for binding any labels will be higher, the more the conditions for removing the protecting groups differ from each other.

The attachment of a label to the deprotected functional group may be attained by compounds inducing condensation of the functional group, at the ligand or the backbone and, for example, a carboxylic acid on the label. Another mode to bind the label is to use a reactive species of that label, like an activated ester. Such reactive species are well known to a man skilled in the art.

A further subject of the present invention is a method of preparing a nucleic acid binding compound (oligomer) being labeled at ligands at two selected portions. This method comprises:
1. providing a protected nucleic acid binding compound (oligomer) comprising a backbone having a plurality of ligands capable of binding to nucleobases of a nucleic acid via hydrogen bonds, wherein at least one of said ligands contains a primary or secondary amino group protected by a first protecting group P1 removable by a medium strong base, and at least one further ligand containing a primary or secondary amino group protected by a second protecting group removable under conditions wherein the first amino protecting group P1 is not removed,
2. removing said second protecting group thereby producing a first unprotected primary or secondary amino group,
3. binding a first label (for example biotin) to said first unprotected amino group,
4. removing said first protecting group P1 thereby producing a second unprotected amino group,
5. binding a second label (for example a ruthenium complex) to said second unprotected amino group.

The compound formed is then, if required, fully deprotected and cleaved from the solid phase on which it was produced.

A further subject of the present invention are compounds of the general formulae IVa, IVb or IVc: 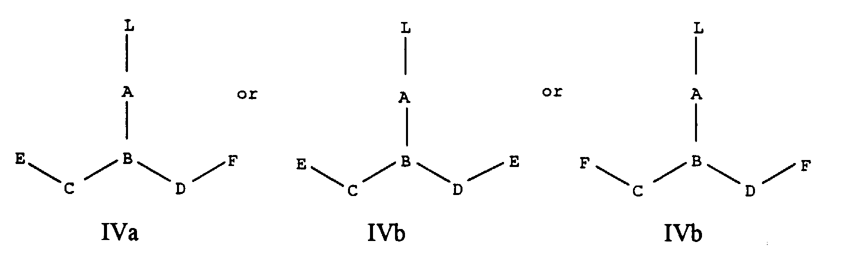 wherein
A, B, C, D, E, and F are defined as for formulae IIIa, IIIb and IIIc, and
L is a group of formula V: 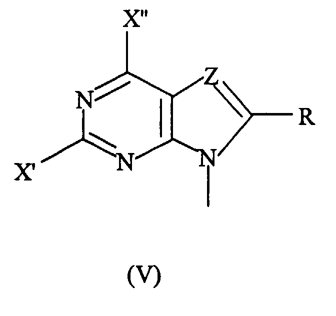 wherein
   X' and X'' are independently selected from O-R' and NH-R'', wherein R' and R'' are protecting groups,
   Z is CH or N and
   R is hydrogen, (C₁ -C₄)-alkyl, aryl, alkyl-aryl, halogen, hydroxyl, alkoxy, or a label as defined above.

In formula V the protecting groups R' and R'' are preferentially different. Thus, R 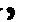 is preferably a medium strong acid removable protecting group like benzyl, and R 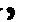 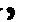 is preferably a medium strong base removable group like acyl or an aroyl group, for example, acetyl, isobutyryl and benzoyl, or R 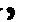 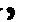 may also be hydrogen.

In the case where both X' and X'' are NH-R'', then R'' can be the same or different. R'' is preferably a group of the formula IV 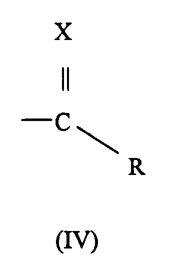 wherein X is O and S, and R is selected from straight or branched chain (C₁ - C₆)alkyl which may be unsubstituted or substituted by C₁-C₄-alkoxy or substituted aromatic moieties, and aromatic moieties containing from 6 to 10 aromatic carbon atoms, which may be unsubstituted or substituted by C₁-C₄-alkyl, C₁-C₄-alkoxy, halogen (preferred Cl) or nitro.

Most preferable the isobutyryl or the acetyl group.

Preferred examples of ligands of general formula V are di/mono-protected diaminopurine and diprotected guanosine.

Advantages of the new strategy can be higher yield of crude product, fast coupling rates of the monomers, and easy large scale production. Further, even chimeric compounds can be prepared. Even very sensitive compounds like S-S containing compounds are preparable using this strategy. The new strategy is further advantageous for the preparation of mixed sequence nucleic binding partners containing A and G or D as bases.

The present invention is exemplified by the following examples:

### Examples

### General

All reagents used are analytical grade and from Aldrich. HATU was purchased from PerSeptive Biosystems (MA). MBHA resin were purchased from NovaBiochem (Switzerland). EZ-Link™NHS-SS-Biotin was from Pierce (IL). Bis-(2,2 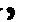 -bipy)4-(4-(4 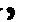 -Me-2,2 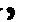 -bipy))-butane Acid-Ruthenium (II)-bis-hexafluoro Phosphate was purchased from Boehringer Mannheim GmbH. N-MMT-(CH2)-aminoModifier was from Clontech Palo Alto(CA,USA). The oligomers used in the examples are linear and single stranded.

For the reactions exemplified in the examples reference is made to the figures showing synthesis flow schemes and numberings for the compounds.

### Abbreviations:

- HOBt:: 1-Hydroxybenzotriazole
- DCM:: Di-chloromethane
- MBHA:: Methyl-benzhydrylamine
- PAM:: Phenylacetamidomethyl
- HATU:: O-(7-azabenzotriazol-1-yl)-1,1,3,3,-tetramethyluronium hexafluorophasphate
- DIEA:: Diisopropylamine
- Ado:: 8-Amino-3,6,-dioxa-octanoic acid

### Example I

### Monomer preparation

### 1-(Benzyloxycarbonylmethyl)-4-amino-2-oxo-pyrimidine (2)

Sodium hydride (8.35 g of a 60 % suspension) was added to a solution of cytosine (**1**) (20 g, 180 mmol) in DMF (400 mL) during magnetic stirring. The mixture was kept at 50 ⁰C for 2 h, after which no further formation of H₂ was seen. After cooling (below 30⁰C), benzyl2-bromoacetate (45.75 g, 31.6 mL, 200 mmol) was added dropwise during 1 h, and the solution was stirred overnight at room temperature. After cooling, the crude product was obtained by filtration and was stirred with a sodium hydrogen carbonate solution (6 %, 400 mL), filtered and washed with water and diethyl ether. Yield: 20 g (43 %).

### 1-(Benzyloxycarbonylmethyl)-N⁴-(benzoyl)-4-amino-2-oxo-pyrimidine (3)

To a suspension of 1-(benzyloxycarbonylmethyl)-4-amino-2-oxo-pyrimidine (**2**) (10.0 g, 38.6 mmol) in pyridine (100 mL) was benzoyl chloride dropwise added (5.2 mL) over 10 min. After standing for 3 h at room temperature, additional benzoyl chloride (1 mL) was added and the mixture was stirred for 1 h. Methanol was added (10 mL) and the solvent was removed by evaporation in vacuo. The crude product was coevaporated with toluene. Recrystallization from ethyl alcohol (approx. 1 L) gave the pure product (**3**). Yield: 12.1 g (86 %).

### 1-(Carboxymethyl)-N4-(benzoyl)-4-amino-2-oxo-pyrimidine (4)

1-(Benzyloxycarbonylmethyl)-N⁴-(benzoyl)-4-amino-2-oxo-pyrimidine (**3**) (9.2 g, 25.3 mmol) was suspended in a mixture of water (400 mL) and THF (50 mL), followed by addition of LiOH (1 M solution, 75 mL, 75 mmol). After 1 h the solution was filtrated and concentrated in vacuo. The crude product was redissolved in water, and the product (**4**) precipitated upon adjusting the pH to 2.8 with aq. 2 N NaHSO₄. Yield: 5.1 g (78 %).

### N-((N⁴-(Benzoyl)-4-amino-2-oxo-pyrimidine-1-yl)acetyl)-N-(2-Boc-aminoethyl)-glycine methyl ester (6)

Boc-backbone (**5**) (1.0 g, 4.3 mmol) was dissolved in DMF (5 mL) and HOBt (670 mg, 5 mmol), 1-(carboxymethyl)-N⁴-benzoyl-4-amino-2-oxo-pyrimidine (**4**) (1.12 g, 4.1 mmol) and DCC (1.0 g, 4.8 mmol) was added. The mixture was stirred for 3 h at room temperature, followed by the addition of DCM (20 mL). The organic phase was filtrated and washed with aq. NaHCO₃ (2x20 mL) and with aq. NaHSO₄ (2x20 ML). After drying over MgSO₄, the crude product (**6**) was isolated by evaporation in vacuo. Yield: 4.55 g (57 %).

### N-((N⁴-(Benzoyl)-4-amino-2-oxo-pyrimidin-1-yl)acetyl)-N-(2-Boc-aminoethyl)-glycine (N⁴-Benzoyl-Boc-C-monomer) (7)

N-((N⁴-(Benzoyl)-4-amino-2-oxo-pyrimidine-1-yl)acetyl)-N-(2-boc-aminoethyl)-glycine methyl ester (**6**) (2.0 g, 4.1 mmol) was dissolved in NaOH (2 M, 10 mL) and after stirring at room temperature for 1 h, the mixture was filtered and pH adjusted to 2.5 with NaHSO₄ (2 M). The crude product was isolated by filtration. The product was dissolved in a minimum of ethyl acetate and was precipitated by dropwise addition to n-hexane. Yield: 1.4 g (72 %).

### 9-(Ethyloxycarbonylmethyl)-6-aminopurine (9)

Adenine (**8**)(50 g, 0.37 mol) was suspended in DMF (750 mL) in a three neck roundbottle flask equipped with mechanical stirring, nitrogen inlet, and a dropping funnel. Sodium hydride (16 g, 0.40 mol, 60% suspension) was added at 10 °C in portions over 1 h. The mixture was stirred at room temperature for 1 h, followed by dropwise addition of 2-bromoacetic acid ethyl ester (123 g, 82 mL, 0.74 mol) over 3 h, and subsequently left at room temperature for 20 h. The yellow solution was evaporated to dryness and stirred for 2 h with water (500 mL). The crude product was collected by filtration, washed with water and cold ethanol. Yield: 36 g (44 %).

### 9-(Ethyloxycarbonylmethyl)-(N⁶-benzoyl)-6-aminopurine (10)

Benzoylchioride (7.27 g, 6.0 mL, 5.17 mmol) was added dropwise at 0 °C to a solution of 9-(ethyloxycarbonylmethyl)-6-aminopurine (**9**) (10 g, 4.5 mmol) in pyridine (150 mL), and stirred over night at room temperature. Methanol (10 mL) was added and the mixture was evaporated to dryness. The crude product was redissolved in hot ethanol (250 mL) and precipitated at 0 °C over night. After filtration and washing with ethanol and diethyl ether the product was isolated. Yield: 9.0 g (62 %).

### 9-(Carboxymethyl)-(N⁶-benzoyl)-6-aminopurine (11)

9-(Ethyloxycarbonylmethyl)-(N⁶-benzoyl)-6-aminopurine (**10**) (9.0 g) was dissolved in water (200 mL), followed by dropwise addition of NaOH (2 M, 60 mL) at 0 °C. After 30 min the temperature was allowed to reach room temperature and the solution was left for 1 h. Then pH was adjusted to 2.5 with NaHSO₄ (2 M), and after cooling (0 °C), the product precipitated, was collected by filtration, and washed with cold water. Yield: 3.5 g (56 %).

### N-((N⁶-(Benzoyl)-6-aminopurine-9-yl)acetyl)-N-(2-boc-aminoethyl)-glycine methyl ester (12)

Boc-backbone (**5**) (1.0 g, 4.3 mmol) was dissolved in DMF (5 mL) and HOBt (670 mg, 5 mmol), 9-(carboxymethyl)-N⁶-benzoyl-6-aminopurine (1.2 g, 4.1 mmol) and DCC (1.0 g, 4.8 mmol) was added. The mixture was stirred for 3 h at room temperature, after which DCM (20 mL) was added and the precipitate was filtered off. The solution was washed with aq. NaHCO₃ (2x20 mL) and with aq. NaHSO₄ (2x20 mL). After drying over MgSO₄, the crude product (5) was isolated by evaporation in vacuo. Yield: 1.4 g (67 %).

### N-((N⁶(Benzoyl)-6-aminopurine-9-yl)acetyl)-N-(2-Boc-aminoethyl)-glycine (N⁶-Benzoyl-Boc-A-monomer) (13)

The crude N-((N⁶-(Benzoyl)-6-aminopurine-9-yl)acetyl)-N-(2-boc-aminoethyl)-glycine methyl ester (**12**) (900 mg) was dissolved in a mixture of THF (2.5 mL) and water (5 mL), followed by addition of LiOH (1 M, 5 mL). After stirring at room temperature for 1 h, the THF was removed by evaporation. The product was precipitated by adjusting the pH to 2.8 with NaHSO₄ (2 N). The crude product was redissolved in minimal amount DCM and precipitated by addition of n-hexane (200 mL).

### 9-(Ethyloxycarbonylmethyl)-2-amino-6-chloropurine (15)

A suspension of 2-amino-6-chloropurine (**14**) (25 g, 143 mmol) in DMF (500 mL), was stirred for 1 h with potassium carbonate (41 g, 0.40 mol). Ethyl 2-bromoacetate (30 g, 20 mL, 180 mmol) was added, and the mixture left at room temperature for 20 h. The resulting mixture was filtered through celite and concentrated in vacuo. The crude product was recrystallized from abs. ethanol. Yield: 25.6 g (70%).

### 9-(Carboxymethyl)-2-amino-6-O-benzylpurine (16)

9-(Ethyloxycarbonylmethyl)-2-amino-6-chloropurine (**15**) (10 g, 39.1 mmol) was added to a mixture of benzyl alcohol (100 mL) and sodium (2.7 g). This gave a thick yellow mixture, which was left over night at room temperature. After addition of water (200 mL), the solution was washed with diethyl ether (2x100 mL). The pH of the water-phase was adjusted to 2 with NaHSO₄ (2 M), and the product was isolated by filtration. The product was recrystallized from ethanol (approx. 1 L). Yield 10.9 g (93%).

### 9-Carboxymethyl-2-isobutyryl-6-O-benzyl-2-aminopurine (17)

9-Carboxymethyl-2-amino-6-O-benzylpurine (**16**) (2.5 g, 8.36 mmol) was dissolved in pyridine (25 mL) and cooled at 0 °C. Isobutyrylchioride (1.5 mL, 14 mmol) was added dropwise over 10 min, and after 2 h, the temperature of the mixture was allowed to reach room temperature and left overnight. The solvent was evaporated in vacuo and the material was resuspended in NaHCO₃ (0.5 M). The solution was filtered and the pH adjusted to 2.5 with 2 N NaHSO₄. The crude product was collected on filtration. Yield 1.6 g (52 %).

### N-((N²-(isobutyryl)-6-O-benzyl-2-aminopurine-9-yl)acetyl)-N-(2-Boc-aminoethyl)-glycine methyl ester (18)

Boc-backbone (**5**) (1.0 g, 4.3 mmol) was dissolved in DMF (5 mL) and HOBt (670 mg, 5 mmol), 9-(carboxymethyl)-2-isobutyryl-6-O-benzyl-2-aminopurine (**17**) (1.5 g, 4.1 mmol) and DCC (1.0 g, 4.8 mmol) was added and the mixture stirred for 3 h at room temperature. The reaction mixture was diluted with DCM (20 mL) and filtrated. The organic phase was washed with aq. NaHCO₃ (2x20 mL) and with aq. NaHSO₄ (2x20 mL). After drying over MgSO₄ the crude product was isolated by evaporation in vacuo. Yield: 850 mg (36%)

### N-((N²-(isobutyryl)-6-O-benzyl-2-aminopurine-9-yl)acetyl)-N-(2-Boc-aminoethyl)-glycine. (N²-(Isobutyryl)-6-O-benzyl-Boc-G-monomer) (19)

Crude N,N-((2-isobutyryl-6-O-benzyl-2-aminopurine-9-yl)acetyl)-(2-boc-aminoethyl) glycine methyl ester (**18**) (400 mg, 0.69 mmol) was dissolved in a mixture of THF (2 mL) and water (2.5 mL) and hydrolyzed by adding LiOH (1 M, 2 mL). After stirring at room temperature for 1 h, the mixture was filtrated and THF was removed by evaporation. Water (10 mL) was added, pH was adjusted to 2.5 with NaHSO₄ (2 N), and the product precipitated. Yield: 350 mg (88.4%).

### 9-(Ethoxycarbonyl)-2,6-diaminopurine (20)

To a suspension of 2,6-diaminopurine (15 g, 100 mmol) in DMF (25 ml) was added K₂CO₃ (41 g, 300 mmol). The suspension was stirred for 1 h at room temperature and added ethyl-2-bromoacetate (13.3 ml 120 mmol). The suspension was stirred for additional 3 h. The reaction mixture was filtered and the collected material was washed with DMF (100 ml). The solid was suspended in abs. ethanol (200 ml) and heated, and the refluxing suspension was subsequently hot filtrated. The filtrate was boiled with activated carbon and filtered through a layer celite. The volume of the filtrate was reduced to 30 ml and cooled and the product precipitated. Yield: 12.5 g (53 %).
¹H NMR, ^{6d}DMSO, 400 MHz: 7.69 ppm (1 H, H-8); 6.72 ppm (2 H, NH₂); 5.83 ppm (2 H, NH₂); 4.85 ppm (2 H, CH₂CO); 4.15 ppm (2 H, OCH₂); 1.2 ppm (3 H, CH₂CH₃).

### 9-(Ethoxycarbonyl)-(N², N⁶-diisobutyryl)-2,6-diaminopurine (21)

A suspension of 9-(ethoxycarbonyl)-2,6-diaminopurine (1.6 g, 6.8 mmol) in pyridine (20 ml) was stirred at 0 °C and isobutyrylchloride (2.9 ml, 27.4 mmol) was added. The mixture was stirred for additional 2 h. The reaction mixture was evaporated to dryness and the residue was redissolved in DCM and extracted with diluted HCl. The organic phase was dried with MgSO₄ and evaporated to dryness. The residue was crystallized from abs. ethanol. Yield 2.35 g (92 %).
Mw. C₁₇H₂₄N₆O₄ Calc./found: 376/377
¹H NMR, ^{6d}DMSO, 400 MHz: 10.48 ppm and 10.99 ppm (1 H, NH); 8.29 ppm (1 H, H-8); 5.09 ppm (2 H, CH₂CO); 4.18 ppm (2H, OCH₂); 3.06 ppm and 2.93 ppm (1H, CH); 1.22 ppm (3 H, CH₂CH₃); 1.10 ppm (3 H, CH₃).

### 9-(Carboxymethyl)-(N²,N⁶-diisobutyryl)-2,6-diaminopurine

9-(Ethoxycarbonyl)-(N², N⁶-diisobutyryl)-2,6-diaminopurine (2.2 g, 5.85 mmol) was stirred for 2 h in water /THF (50 ml, 2:1) and LiOH (1 M, 16 ml) was added. The solid was soon dissolved. The solution was adjusted to pH 2.5 with NaHSO₄ (2 M) and subsequently extracted with DCM (2 x 50 ml). The organic phases were combined, extracted with brine, and dried with MgSO₄. The solvent was removed by evaporation. Recrystallisation from abs. ethanol. Yield: 1.9 g , (95.5%).
MW. C₁₅H₂₀N₆O₄ Calc./found: 348/349

### N-((N²,N⁶⁻Diisobutyryl-2,6-diaminopurine-9-yl)acetyl)-N-(-2-Boc-aminoethyl)-glycine. (N²,N⁶-diisobutyryl-Boc-D-Monomer) (22)

9-(Carboxymethyl)-(N²,N⁶-diisobutyryl)-2,6-diaminopurine (1.5 g, 4.4 mmol), DCC (1 g, 4.8 mmol), HOBT (0.66 g, 4,8 mmol) and methyl N-Boc-aminoethyl glycinate (1.23 g, 5.3 mmol) was stirred in DMF (30 ml) over night. The reaction mixture was filtered and the collected material was washed with DCM (2 x 30 ml). The filtrate was extracted with diluted NaHCO₃ (3 x 20 ml), NaHSO₄ (2 M, 2 x 20 ml) and brine (30 ml). The organic phase was dried with MgSO₄ and evaporated to dryness. Recrystallization from ethanol gave N-(N²,N⁶-diisobuturyl-2,6-diaminopurine-9-yl)acetyl)-N-(Boc-aminoethyl) methyl glycinate. Yield: 2.2 g, (90 %).
MW. C₂₅H₃₈N₈O₇ Calc./found: 562/562.

Without further purification N-(N²,N⁶-diisobuturyl-2,6-diaminopurine-9-yl)acetyl)-N-(Boc-aminoethyl) methyl glycinate (2.0 g, 3.6 mmol) was suspended in water/THF (40 ml, 10/1) LiOH (1 M, 6 ml) was added and the compound went slowly into solution. The solution was adjusted to pH 2.8 with NaHSO₄ (2 M) and extracted with DCM (2 x 30 ml). The organic phase was dried with MgSO₄ and evaporated to dryness. The product was recrystallized from ethanol. Yield 1.8 g, (92 %).
¹H NMR ^{6d}DMSO 400 MHz: 10.51 ppm(s, 1H, NH); 10.32 ppm (s 1, H, NH); 8.16 ppm and 8.15 ppm (s 1, H, H-8); 7.04 ppm and 6.76 ppm (m, 1 H, BocHN); 5.21 ppm and 5.10 ppm (s, 2 H, CH₂CO); 4.35 ppm and 4.00 ppm(s, 2H, CH₂CO); 3.1 ppm (m, 4H, CH₂); 1.35 ppm and 1.33 ppm (s, 9 H, Boc); 1.1 ppm (m, 12 H, isobut.).
Mw. C₂₄ H₃₆N₈O₇, Calc./found: 548/548

Mono-isobutyryl protected diamino purine was prepared from **22** by treatment with 10 % ammonia in water at room temperature for 1 h.

### Synthesis of Boc-Tlys(Fmoc)-OH

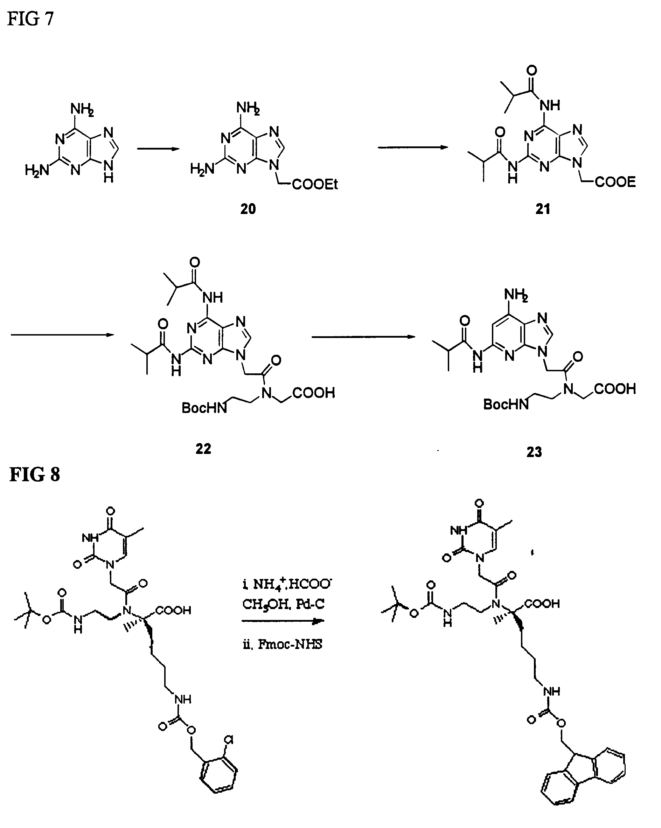

### Nα- (2-tert.-butoxycarbonylaminoethyl)-Nα-(1-thyminylaceryl)-Nε-(9-fluorenylmethyloxycarbonyl)-D-lysine (FIG. 8)

*Nα*- (2-tert-butyloxycarbonylaminoethyl)-*Nα*-(1-thyminylacetyl)-*Nε*-(2-chlorobenzyloxycarbonyl)-D-lysine (1 g, 1.6 mmol) was dissolved in a solution of ammoniumformiate 4.4 % in methanol (20 ml). To the solution was added Pd-black (200 mg) and the suspension was stirred for 1 h. The suspension was filtered through Celite and evaporated to dryness. The solid was dissolved in DMF (25 ml), DIEA (0.4 ml) and Fmoc-N-hydroxy-succinimide (800 mg) was added and the mixture was stirred for 2 h. The reaction mixture was evaporated and extracted with sodium hydrogen carbonate (50 ml, 0.5 M). The water phase was filtered and pH was adjusted to 2.8 by adding sodium hydrogen sulfate (2 M) whereby the target molecule precipitated. The precipitate was dissolved in ethylacetate (5 ml) and to the solution was added hexane (100 ml) dropwise whereby the target molecule precipitated. The product was filtered and washed with hexane.
Yield: 885 mg (1.3 mmol, 75 %).
¹H-NMR (DMSO-d₆/TMS): δ= 1.23 (m, 7 H, CH₂), 1.35 and 1.38 (s, 9 H, Boc), 1.73 and 1.75 (s, 9 H, Boc), 3.00 (m, 2 H, CH₂), 3.21 (m, 2 H, CH₂), 4.27 (m, 2 H, CH₂CO), 4.64 (m, 1H, CHCO), 6,83 and 6.88 (m, 1 H, BocNH), 7.24-7.43 (m, 9 H, aromatic), 7.66 and 7.69 (d. 1 H, 6-H), 7.87 and 7.90 (d, 1 H, CON), 11.26 and 11.28 (d, 1 H, imide NH).
MS (MALDI-TOF) m/z: 716 (M + K).

### Example 2

### Oligomer (PNA) synthesis

The PNA was assembled using a standard synthesis cycle on a preloaded MBHA support. Preloading was performed with a T-monomer, as previously described (WO92/20702 and Christensen et al., (1995) J.pep.sci., 3, 175-183). The pre-activation time with HATU of the monomers was extended from one to five minutes. In the first coupling Boc-Gly-PAM was linked to the T-resin . All PNA oligomers were acetylated in the final step, to prevent rearrangement of the final monomer during basic deprotection and removal from the support. The oligomerizations of PNA monomers are performed on an ABI 433A peptide synthesizer with software modified to run the PNA synthesis steps. The PNA monomers are pre-dissolved and pipetted into individual cartridges, i.e. one for every coupling.

The reaction scale is 5 µmol and the total reaction volume is 440 µl. A 3 ml reaction vessel is used and the measuring loop has a volume of 150 µl. TFA/m-cresol (95/5; 2 × 180 s) is used to deprotect the Boc protecting group. Between the deprotection and the coupling modules 2 washing modules are inserted, 1 DCM and 1 NMP module, which each consists of 5 consecutive washes. In each coupling 140 µl 0.26 M monomer (36 µmol; in NMP) 150 µl 0.21M HATU (32 µmol; in NMP) and 150 µl 0.50 M DIEA (75 µmol; in pyridine) are delivered. The monomers are pre-activated in the synthesis cartridge for 1 min. Resin neutralization and addition of activated monomer are performed simultaneously. The coupling time is 10 min and PNA monomers are used in a 7 fold excess over the initial substitution of the resin. During the coupling the monomer concentration is 0.08 M. Capping (acetic anhydride/NMP/pyridine (1:25/25), is performed for 1 min.

### Summary of the synthesis cycle

1. Removal of Boc-group (2x3 min)
2. Wash (DCM and DMF)
3. Coupling in 15 min
4. Wash (DCM and DMF)
5. Capping (1 min)
6. Wash (DCM and DMF)

Unless otherwise stated, reaction yields are assessed by quantitative Kaiser test. Product yields of oligomers and average coupling yields during oligomerization are determined by HPLC analysis and/or by OD measuring at 260 nm.

Mass spectra are recorded on a Hewlett-Packard MALDI-TOF mass spectrometer (G2025A). The matrix is composed of sinapinic acid (100 mM; in acetonitrile/methanol/water (60/32/8) mixed in the ratio 1:1 with PNA solutions ranging from 10 nM - 100 µM.

Analytical RP-HPLC of the PNA oligomers is performed at 50 °C on a Deltapak (C₁₈, 100Å, 5µm, 3.9 × 150, 1 ml/min (Waters)) with a Hewlett-Packard HPLC system (1050). Two eluents are used: **A**, 0.1 % TFA in water and **B**, 0.1 % TFA in acetonitrile. A linear gradient of 0-40 % of **B** in 30 min is used and eluent is monitored at 260 nm. Cleavage of the protected PNA oligomer from the resin is performed as described earlier.

### Example 3

### Synthesis of oligomers having a PNA and an oligonucleotide part (chimeric molecules; chimera)

The DNA part was synthesized using standard phosphoramidite synthesis on a CPG support. The introduction of the 5 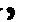 -amino functionality, was achieved using a special 6-(Mmt-amino)hexyl phosphoramidite. The support was transferred to a reaction vessel, and the Mmt group was removed during treatment with 3% trichloroacetic acid in DCM. The PNA monomers were added using the above described cycle. The synthesis was also achieved on a ABI 433A peptide synthesizer using methods in which the vortex has been exchanged with nitrogen purging, to be able to use DNA on CPG resin.

Ruthenium complexes were coupled to the N-terminus of the distal PNA monomer by activation of the carboxylic group on the Ruthenium conjugand by HATU, biotin was also activated by HATU but double coupled, fluoresceine isothiocyanate was coupled for 24 h without further activation and with only catalytic amount of DIEA. When biotin or fluorescein was coupled, the final PNA monomer was not capped with acetic anhydride.

The final chimera was removed from the support and deprotected using either ammonia in water (32%) for 20 h at 60 °C or with methylamine (40%) at room temperature for 4 h.

### Synthesis of Ac-TGT-ACG-TCA-CAA-CTA-Gly

The PNA synthesis was essentially performed as described above.

The total yield was estimated by redissolving the crude product in 5% acetic acid and measuring the UV absorption at 260 nm, and by using the standard values for absorption from the four nucleobases normally used for DNA. Purity as measured by HPLC of the crude product was 90.7 %. The total recovery of crude material measured by OD was 68 %.

### Chimeras:

The following chimeras have been synthesized using the above described method (PNA in italic)
A: Fluoreseine-*TAAATCAGCTC*A-NH-(CH₂)₆-TTTTT
B: Biotin-*CCTGATAGACGG*-NH-(CH₂)₆-TTTTT
C: Biotin-*TGTTAAATCAGCTCA*-NH-(CH₂)₆-TTTTT
D: Ruthenium- *TGTTAAATCAGCTCA*-NH-(CH₂)₆-TTTTT

C and D were also synthesized using the ABI 433A peptide synthesizer, using a method in which all vortex steps have been exchanged with nitrogen purging. The purity of all chimeras were checked by HPLC and the structure confirmed by MALDI-TOF mass spectroscopy.

Regarding chimeric molecules it is advisable to produce the PNA segment first, or to chose the sequence such that the first produced DNA segment only contains pyrimidine bases, whereas the subsequently produced PNA segment can have any chosen bases.

### Boc-Gly-PAM:

To a solution of Boc-glycine (1.12 g, 6.4 mmol) dissolved in DMF (5 ml) was added 4-bromomethylphenyl acetic acid phenacyl ester (1.5 g, 4.3 mmol) dissolved in acetonitrile (30 ml), followed by addition of potassium fluoride dihydrate (900 mg, 9.56 mmol). The mixture was stirred at 50 °C for 20 h. The solid inorganic material was removed by filtration and ethyl acetate (60 ml) and water (60 ml) was added. The aqueous layer was extracted with ethyl acetate (3x50 ml) and the combined organic phases were washed with water (3x50 ml). Excess Boc-glycine was removed by washing with a buffer solution (3x100 ml)(6.9 g K₂CO₃ and 8.4 g NaHCO₃ in 300 ml water). Alter drying over magnesium sulfate the solvent was removed by rotary evaporation, to give the product as a yellow solid. Yield = 1.7 g (90%).

The product (1.6 g, 3.6 mmol) was dissolved in acetic acid (40 ml) and water (85:15) followed by addition of zinc dust (5 g). The mixture was stirred at room temperature for 4 hours. After filtration, ethyl acetate (150 ml) and water (150 ml) was added, and the water phase was made acidic (pH ∼ 1.5) by addition of aqueous HCl (0.5 M). The water phase was extracted with ethyl acetate (3x100 ml) and the combined organic phases were washed with water (10x50 ml). The crude product was obtained after drying over magnesium sulfate and concentration using rotary evaporator. The crude product was redissolved in a minimum of ethyl acetate and precipitated by dropwise addition to n-hexane. This gave 850 mg (73%) of the pure product as seen by HPLC.

### Synthesis of Bio-S-S-Lys-(T)₇-Ado-Lys-Ado-Lys-Ado-(T)₇-Ado-Gly-NHCH₃

Synthesis of Bio-S-S- lys- Ado- TTT TTT T-Ado- Lys - Ado - Lys - Ado -T TTT TTT-Ado - GlyNHCH₃ (5 µmoles) was done on HMBA support preloaded with a T - monomer as described above. The base labile linker Boc-Gly-PAM was coupled as the first residue. Ado- and T - monomers were Boc protected and lysine was α-Boc, γ-Fmoc protected. The S-S-Biotin moiety was coupled by reacting N-hydroxysulfo-succinimide ester (EZ-Link, 10 mg, 16.5 µmole) with the terminal Boc- deprotected lysine. The reaction was done for 1 h in NMP with added DIEA to keep the conditions basic. The PNA was cleaved from the resin and the Fmoc-lysine deprotected by treatment with 40 % methylamine in water for 0.5 h.

This protection and cleavage strategy provides a direct conjugation of the -S-S- moiety to the PNA on the resin.

## Claims

1. A method for the preparation of a nucleic acid binding compound comprising the steps of
a) preparing a protected nucleic acid binding compound comprising a plurality of ligands bound to a backbone capable of binding to nucleobases of a nucleic acid via hydrogen bonds, at least one of said ligands containing a primary or secondary amino group protected by a first protecting group P1 removable by a medium strong base, said backbone further comprising a protected amino group of the formula O: 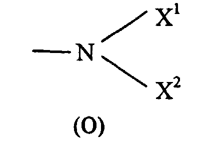 wherein X¹ and is selected from hydrogen, (C₁ - C₃)-alkyl and an amino-protecting group P2 removable by a medium strong acid and X² is an amino protecting group P3 removable by a medium strong acid;
b) removing said medium strong acid removable group(s); and
c) removing said medium strong base removable group(s).

2. A method according to claim 1 wherein said medium strong acid removable group is an amino-protecting group of the urethane type.

3. A method according to claim 2 wherein said urethane type group is the Boc group.

4. A method according to any of the proceeding claims, wherein said first group is a group of the formula IV 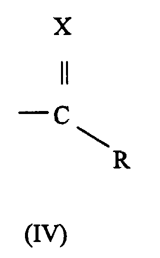 wherein
X is selected from O and S and
R is selected from straight or branched chain (C₁ - C₆)-alkyl, which may be unsubstituted or substituted by C₁-C₄-alkoxy or substituted aromatic moieties, and aromatic moieties containing from 6 to 10 aromatic carbon atoms, which may be unsubstituted or substituted by C₁-C₄-alkyl, C₁-C₄-alkoxy, halogen or nitro.

5. A method according to claim 1, wherein said nucleic acid binding compound in protected form further contains a ligand containing a primary or secondary amino group protected by a second group removable under conditions wherein none of or not both of the other amino-protecting groups are removed.

6. A method according to claim 5, wherein the first and second groups are bound to a base of the same type, but at different positions.

7. A method according to claim 1, wherein said compound is bound to a solid phase.

8. A compound of the general formula IIIa, IIIb or IIIc 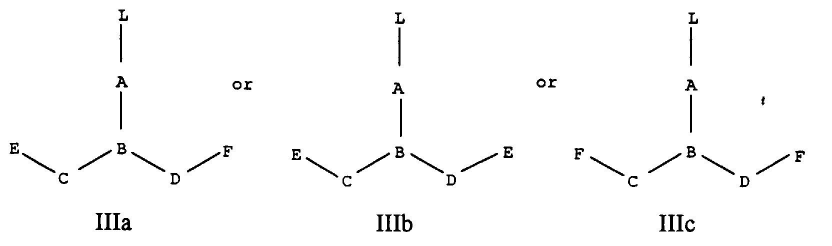 wherein
A is chosen from the definitions of A¹-Aⁿ in formula I,
B is chosen from the definitions of B¹-Bⁿ in formula I,
C is chosen from the definitions of C¹-Cⁿ in formula I,
D is chosen from the definitions of D¹-Dⁿ in formula I,
E is COOH, CSOH, SOOH, SO₂OH or an activated derivative thereof,
and L is a group of formula V 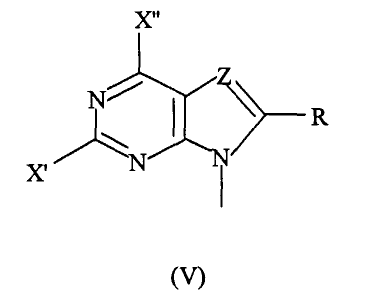 wherein
X' and X'' are independently selected from O-R' and NH-R'', wherein
R' and R'' are protecting groups,
Z is CH or N, and
R is hydrogen, (C₁ -C₄)-alkyl, aryl, alkyl-aryl, halogen, hydroxyl, alkoxy, or a label.

9. A method of preparing a nucleic acid binding compound being labeled at ligands at two selected portions comprising,
- providing a protected nucleic acid binding compound comprising bound to a backbone a plurality of ligands capable of binding to nucleobases of a nucleic acid via hydrogen bonds, wherein at least one of said ligands contains a primary or secondary amino group protected by a first protecting group P1 removable by a medium strong base and at least one further ligand contains a primary or secondary amino group protected by a second protecting group removable under conditions wherein more of or not both of the other amino protecting groups are removed;
- removing said second group producing a first protected amino group;
- binding a first label to the first unprotected amino group;
- removing said first group producing a second unprotected amino group;
- binding a second label to the second unprotected amino group.
